# EUROPEAN PATENT APPLICATION

(11) **EP 4 156 203 A1**
(43) Date of publication of application: **29.03.2023**
(21) Application number: 22197572.5
(22) Date of filing: 23.09.2022
(51) Int. Cl.: G16H 40/40, G16H 40/60

(54) **SYSTEMS AND METHODS FOR DEVICE PARAMETER CONFIGURATION**

(30) Priority: 24.09.2021 CN 202111122191
(71) Applicant: Wuhan United Imaging Healthcare Co., Ltd., WuHan, Hubei 430206 (CN)
(72) Inventor: YE, Jialin, Wuhan, Hubei, 430206 (CN)
(74) Representative: Wang, Bo

(57) **Abstract**

The present disclosure may provide systems and methods for device parameter configuration. The systems may obtain a request for parameter configuration of a device. In response to receiving the request, the systems may acquire, from a first storage device, log data of the device. The log data may include data of one or more actual parameters indicating an operation of the device. The systems may also acquire, from a second storage device, data of one or more reference parameters of the device. The systems may obtain a comparison result by comparing the data of the one or more actual parameters and the data of the one or more reference parameters of the device. The systems may generate a feedback based on the comparison result.

## Description

### TECHNICAL FIELD

The present disclosure generally relates to device monitoring, and in particular, to systems and methods for parameter configuration of a device.

### BACKGROUND

An operation of a device (e.g., a magnetic resonance imaging (MRI) device) may be monitored by analyzing one or more parameters of the device in log data of the device. Therefore, it is desirable to provide systems and methods for efficiently configuring one or more parameters used to monitor an operation of a device.

### SUMMARY

According to one aspect of the present disclosure, a method may be provided. The method may be implemented on a computing device having at least one processor and at least one storage device. The method may include: obtaining a request for parameter configuration of a device; characterized by further comprising: in response to receiving the request, acquiring, from a first storage device, log data of the device, the log data including data of one or more actual parameters indicating an operation of the device; acquiring, from a second storage device, data of one or more reference parameters of the device; obtaining a comparison result by comparing the data of the one or more actual parameters and the data of the one or more reference parameters of the device; and generating a feedback based on the comparison result.

In some embodiments, the second storage device may store a corresponding relationship between an identity of each of multiple devices and data of reference parameters of the each of the multiple devices. The acquiring the data of the one or more reference parameters of the device may include: obtaining an identity of the device; obtaining the corresponding relationship from the second storage device; and determining the data of the one or more reference parameters based on the identity of the device and the corresponding relationship.

In some embodiments, the second storage device may store a corresponding relationship between multiple devices in the same type and data of reference parameters of the multiple devices in the same type. The acquiring the data of the one or more reference parameters of the device may include: determining a type of the device; obtaining the corresponding relationship from the second storage device; and determining the data of the one or more reference parameters based on the type of the device and the corresponding relationship.

In some embodiments, the second storage device may store a corresponding relationship between feature information associated with each of multiple devices and data of reference parameters of the each of the multiple devices. The acquiring the data of the one or more reference parameters of the device may include: obtaining feature information associated with the device; obtaining the corresponding relationship from the second storage device; and determining the data of the one or more reference parameters based on the feature information associated with the device and the corresponding relationship.

In some embodiments, the corresponding relationship may be implemented by a parameter model. The determining the data of the one or more reference parameters based on the feature information associated with the device and the corresponding relationship may include: determining the data of the one or more reference parameters by inputting the feature information associated with the device into the parameter model.

In some embodiments, the obtaining the comparison result by comparing the data of the one or more actual parameters and the data of the one or more reference parameters of the device may include: determining a difference between the one or more actual parameters and the one or more reference parameters, the difference including at least one of a type difference or a magnitude difference.

In some embodiments, the generating the feedback based on the comparison result may include: in response to determining that the comparison result indicates that the difference between the one or more actual parameters and the one or more reference parameters satisfies a condition, determining the feedback including adjusting the data of the one or more reference parameters of the device based on the difference between the one or more actual parameters and the one or more predetermined parameters.

In some embodiments, the difference between the one or more actual parameters and the one or more reference parameters satisfying the condition may include at least one of a first case or a second case, in the first case, a type of an actual parameter may be different from each type of the one or more reference parameters, and in the second case, a type of a reference parameter may be different from each type of the one or more actual parameters.

In some embodiments, the adjusting the data of the one or more reference parameters of the device based on the difference between the one or more actual parameters and the one or more predetermined parameters may include: updating the data of the one or more reference parameters by adding data of the actual parameter whose type is different from each type of the one or more reference parameters into the data of the one or more reference parameters; or updating the data of the one or more reference parameters by removing the reference parameter whose type is different from each type of the one or more actual parameters.

In some embodiments, the generating the feedback based on the comparison result may include: in response to determining that the comparison result indicates that the difference between the one or more actual parameters and the one or more reference parameters satisfies a condition, determining the feedback including a reminder that the operation of the device includes an anomaly.

In some embodiments, the generating the feedback based on the comparison result may include: in response to determining that the comparison result indicates that the difference between the one or more actual parameters and the one or more reference parameter does not satisfy a condition, determining the feedback including the data of the one or more reference parameters. The method may further include: monitoring the device based on the data of the one or more reference parameters.

In some embodiments, the first storage device may include at least one of a first database or a second database. The first database may be used to store latest log data of the device. The second database may be used to store log data of the device generated at a time period.

In some embodiments, the acquiring the log data of the device may include: acquiring the log data of the device from the first database or the second database, the log data includes latest log data at a current time period.

According to another aspect of the present disclosure, an apparatus may be provided. The apparatus may include at least one storage device including a set of instructions, and at least one processor in communication with the at least one storage device, wherein when executing the set of instructions, the at least one processor may be configured to perform a method. The method may include: obtaining a request for parameter configuration of a device; in response to receiving the request, acquiring, from a first storage device, log data of the device, the log data including data of one or more actual parameters indicating an operation of the device; acquiring, from a second storage device, data of one or more reference parameters of the device; obtaining a comparison result by comparing the data of the one or more actual parameters and the data of the one or more reference parameters of the device; and generating a feedback based on the comparison result.

According to another aspect of the present disclosure, a non-transitory computer readable medium may be provided. The non-transitory computer readable medium may include at least one set of instructions, wherein when executed by at least one processor of a computing device, the at least one set of instructions may cause the at least one processor to effectuate a method comprising: obtaining a request for parameter configuration of a device; in response to receiving the request, acquiring, from a first storage device, log data of the device, the log data including data of one or more actual parameters indicating an operation of the device; acquiring, from a second storage device, data of one or more reference parameters of the device; obtaining a comparison result by comparing the data of the one or more actual parameters and the data of the one or more reference parameters of the device; and generating a feedback based on the comparison result.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities and combinations set forth in the detailed examples discussed below.

### BRIEF DESCRIPTION OF THE DRAWINGS

The present disclosure is further described in terms of exemplary embodiments. These exemplary embodiments are described in detail with reference to the drawings. The drawings are not to scale. These embodiments are non-limiting schematic embodiments, in which like reference numerals represent similar structures throughout the several views of the drawings, and wherein:
FIG. 1 is a schematic diagram illustrating an exemplary parameter configuration system according to some embodiments of the present disclosure;
FIG. 2 is a schematic diagram illustrating exemplary hardware and/or software components of an exemplary computing device according to some embodiments of the present disclosure;
FIG. 3 is a schematic diagram illustrating exemplary hardware and/or software components of an exemplary mobile device according to some embodiments of the present disclosure;
FIG. 4 is a block diagram illustrating an exemplary processing device according to some embodiments of the present disclosure;
FIG. 5 is a flowchart illustrating an exemplary process for parameter configuration according to some embodiments of the present disclosure; and
FIG. 6 is a schematic diagram illustrating exemplary parameter configuration of a device according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high-level, without detail, in order to avoid unnecessarily obscuring aspects of the present disclosure. Various modifications to the disclosed embodiments will be readily apparent to those skilled in the art, and the general principles defined herein may be applied to other embodiments and applications without departing from the spirit and scope of the present disclosure. Thus, the present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be understood that the terms "system," "unit," "module," and/or "block" used herein are one method to distinguish different components, elements, parts, sections or assemblies of different levels in ascending order. However, the terms may be displaced by another expression if they achieve the same purpose.

The modules (or units, blocks, units) described in the present disclosure may be implemented as software and/or hardware modules and may be stored in any type of non-transitory computer-readable medium or other storage devices. In some embodiments, a software module may be compiled and linked into an executable program. It will be appreciated that software modules can be callable from other modules or from themselves, and/or can be invoked in response to detected events or interrupts. Software modules configured for execution on computing devices can be provided on a computer readable medium or as a digital download (and can be originally stored in a compressed or installable format that requires installation, decompression, or decryption prior to execution). Such software code can be stored, partially or fully, on a memory device of the executing computing device, for execution by the computing device. Software instructions can be embedded in a firmware, such as an EPROM. It will be further appreciated that hardware modules (e.g., circuits) can be included in connected or coupled logic units, such as gates and flip-flops, and/or can be included in programmable units, such as programmable gate arrays or processors. The modules or computing device functionality described herein are preferably implemented as hardware modules, but can be software modules as well. In general, the modules described herein refer to logical modules that can be combined with other modules or divided into units despite their physical organization or storage.

Certain terminology has been used to describe embodiments of the present disclosure. For example, the terms "one embodiment," "an embodiment," and/or "some embodiments" mean that a particular feature, structure or characteristic described in connection with the embodiment is in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure.

The flowcharts used in the present disclosure illustrate operations that systems implement according to some embodiments of the present disclosure. It is to be expressly understood, the operations of the flowcharts may be implemented not in order. Conversely, the operations may be implemented in inverted order, or simultaneously. Moreover, one or more other operations may be added to the flowcharts. One or more operations may be removed from the flowcharts.

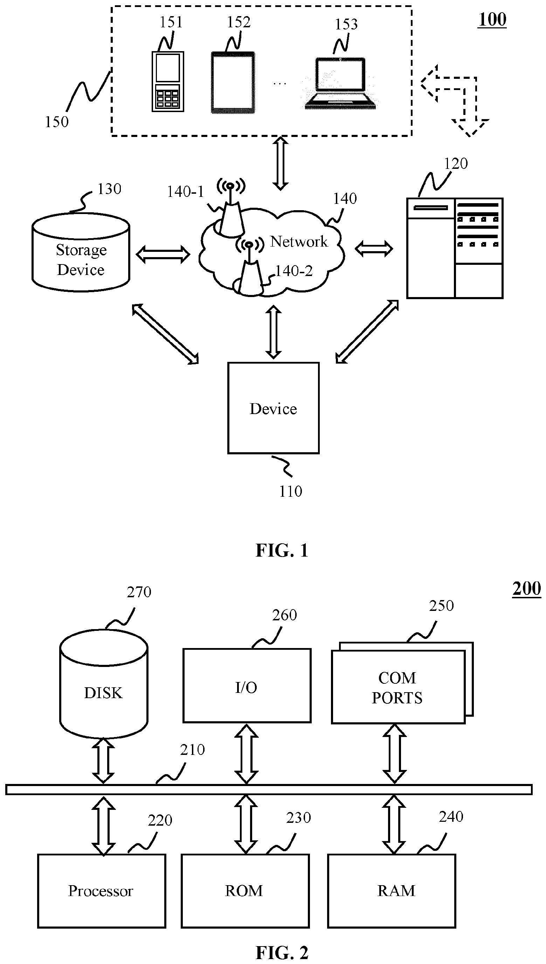

FIG. 1 is a schematic diagram illustrating an exemplary parameter configuration system according to some embodiments of the present disclosure. In some embodiments, the parameter configuration system 100 may include a device 110, a processing device 120, a storage device 130, a network 140, and a terminal device 150.

The parameter configuration system 100 may be configured to facilitate parameter configuration of the device 110. The parameter configuration may include configuring one or more parameters used to facilitate monitoring an operation of the device (also referred to as monitoring parameters or configuration parameters). For example, the one or more monitoring parameters may be used to determine whether the operation of the device is normal.

In some embodiments, the device 110 may include a medical device, a metallurgical device, a chemical device, etc. The medical device may be configured to acquire imaging data of an object (e.g., a patient) by scanning the object and/or facilitate radiation treatment of the object. For example, the medical device may include an imaging device (e.g., a magnetic resonance imaging (MRI) device, an X-ray device, a computed tomography (CT) device, a positron emission tomography (PET) device, a single photon emission computed tomography (SPECT) device), a treatment device (e.g., a linear accelerator, a cyclotron, a synchrotron), or the like, or any combination thereof.

In some embodiments, the one or more parameters may include parameters of at least one component of the device 110. In some embodiments, the device 110 may include an imaging device, and the at least one component may include an imaging source, a detector, a cooling component, a target, a magnet, etc. In some embodiments, the device 110 may include a treatment device, and the at least one component may include a treatment source, a detector (e.g., a electronic portal imaging device (EPID), a collimator, etc. In some embodiments, the device 110 may include a CT imaging device and exemplary parameters may include a parameter of an X-ray tube, a parameter of a target, a parameter of a detector, etc., of the CT imaging device.

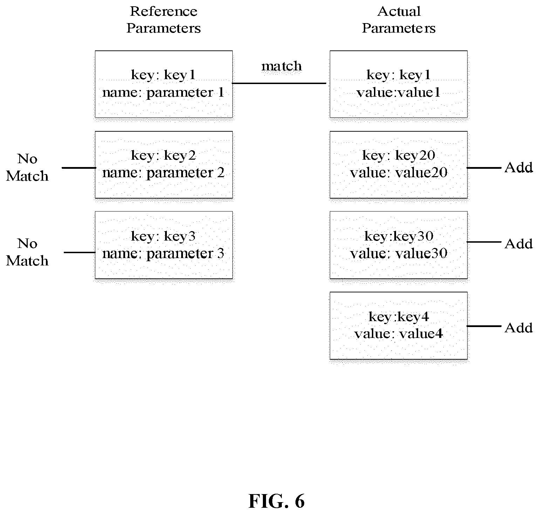

In some embodiments, the device 110 may include an MRI device and exemplary parameters (e.g., as shown in FIG. 6) may include a cooling parameter, a gradient parameter (e.g., a temperature gradient), a magnet parameter (e.g., an intensity), a scanned portion of an object, a remaining disk capacity, or the like, or any combination thereof. In some embodiments, the magnet parameter may be of a main magnet used to generate a main field, be of a gradient coil used to generate a gradient field, be of a radio frequency (RF) coil used to generate an RF field, or the like, or any combination thereof. The cooling parameter may be of a cooling medium (e.g., helium) that is used to cool at least a portion (e.g., a main magnet) of the MRI device. For example, the cooling parameter may include a pressure, a flux, a temperature, etc., of the cooling medium. The gradient parameter may include a temperature gradient of at least a portion (e.g., a main magnet) of the MRI device.

In some embodiments, the device 110 may communicate with one or more components of the parameter configuration system 100 (e.g., the processing device 120, the storage device 130, the terminal device 150) via the network 140. In some embodiments, the device 110 may be directly connected to one or more components of the parameter configuration system 100 (e.g., the processing device 120, the storage device 130, the terminal device 150).

In some embodiments, the processing device 120 may include a single server or a server group. The server group may be centralized or distributed (e.g., the processing device 120 may be a distributed system). In some embodiments, the processing device 120 may be local or remote. For example, the processing device 120 may access information and/or data stored in the device 110 and/or the storage device 130 via the network 140. As another example, the processing device 120 may be directly connected to the device 110 and/or the storage device 130 to access stored information and/or data. In some embodiments, the processing device 120 may be implemented on a cloud platform or an onboard computer. In some embodiments, the processing device 120 may be implemented on a computing device 200 including one or more components illustrated in FIG. 2 of the present disclosure.

In some embodiments, the processing device 120 may process information and/or data associated with parameter configuration to perform one or more functions described in the present disclosure. For example, the processing device 120 may obtain a comparison result by comparing data of one or more actual parameters of the device 110 and data of one or more reference parameters of the device 110. The processing device 120 may also generate a feedback based on the comparison result. In some embodiments, the processing device 120 may include one or more processing engines (e.g., single-core processing engine(s) or multi-core processor(s)). In some embodiments, the processing device 120 may include a Solar network management system.

The storage device 130 may store data and/or instructions. In some embodiments, the storage device 130 may store data obtained from the device 110, the processing device 120, the terminal device 150, or an external storage device. For example, the storage device 130 may store log data of the device 110. In some embodiments, the storage device 130 may store data and/or instructions that the storage device 130 may execute or use to perform exemplary methods described in the present disclosure. For example, the storage device 130 may store instructions that the storage device 130 may execute or use to obtain a comparison result by comparing data of one or more actual parameters of the device 110 and data of one or more reference parameters of the device 110.

In some embodiments, the storage device 130 may include a mass storage, a removable storage, a volatile read-and-write memory, a read-only memory (ROM), or the like, or any combination thereof. In some embodiments, the storage device 130 may be connected to the network 140 to communicate with one or more components (e.g., the device 110, the processing device 120, the terminal device 150) of the parameter configuration system 100. One or more components of the parameter configuration system 100 may access the data or instructions stored in the storage device 130 via the network 140. In some embodiments, the storage device 130 may be directly connected to or communicate with one or more components (e.g., the device 110, the processing device 120, the terminal device 150) of the parameter configuration system 100. In some embodiments, the storage device 130 may be part of the processing device 120. For example, the storage device 130 may be integrated into the processing device 120.

The network 140 may facilitate exchange of information and/or data. In some embodiments, one or more components (e.g., the device 110, the processing device 120, the storage device 130, the terminal device 150) of the parameter configuration system 100 may transmit information and/or data to other component(s) of the parameter configuration system 100 via the network 140. For example, the storage device 130 may acquire log data of the device 110 from the device 110 via the network 140. In some embodiments, the network 140 may be any type of wired or wireless network, or a combination thereof. In some embodiments, the network 140 may include one or more network access points. For example, the network 140 may include wired or wireless network access points (e.g., a point 140-1, a point 140-2), through which one or more components of the parameter configuration system 100 may be connected to the network 140 to exchange data and/or information.

The terminal device 150 may include a mobile device 151, a tablet computer 152, a laptop computer 153, or the like, or any combination thereof. In some embodiments, the mobile device 151 may include a smart home device, a wearable device, a smart mobile device, a virtual reality device, an augmented reality device, or the like, or any combination thereof. In some embodiments, the terminal device 150 may remotely operate the device 110 and/or the processing device 120. In some embodiments, the terminal device 150 may operate the device 110 and/or the processing device 120 via a wireless connection. In some embodiments, the terminal device 150 may receive information and/or instructions inputted by a user, and send the received information and/or instructions to the device 110 or to the processing device 120 via the network 140. For example, the terminal device 150 may transmit a request for parameter configuration of the device 110, update one or more reference parameters of the device 110, display information (e.g., anomaly information of the device 110), receive a feedback, etc. In some embodiments, the terminal device 150 may receive data and/or information from the device 110, the processing device 120, and/or the storage device 130. In some embodiments, the terminal device 150 may be part of the processing device 120. In some embodiments, the terminal device 150 may be omitted.

In some embodiments, two or more components of the parameter configuration system 100 may be integrated in a same device. For example, the processing device 120 and the storage device 130 may be integrated in the same device.

It should be noted that the parameter configuration system 100 is merely provided for the purposes of illustration, and is not intended to limit the scope of the present disclosure. For persons having ordinary skills in the art, multiple variations or modifications may be made under the teachings of the present disclosure. However, those variations and modifications do not depart from the scope of the present disclosure.

In some embodiments, the parameter configuration system 100 may also include a user device (not shown) configured to receive information and/or data from the device 110, the processing device 120, the storage device 130, and/or the terminal device 150. The user device may provide a user interface via which a user may view information (e.g., actual parameters, reference parameters) and/or input data (e.g., request for parameter configuration) and/or instructions to the parameter configuration system 100.

In some embodiments, the parameter configuration system 100 may also include an input/output (I/O) component. The I/O component may be configured to input or output signals, data, or information. In some embodiments, the I/O component may include an input device and an output device. Exemplary input devices may include a keyboard, a mouse, a touch screen, a microphone, or the like, or any combination thereof. Exemplary output devices may include a display device, a speaker, a printer, a projector, or the like, or any combination thereof.

FIG. 2 is a schematic diagram illustrating exemplary hardware and/or software components of an exemplary computing device according to some embodiments of the present disclosure. The computing device 200 may be used to implement any component of the parameter configuration system 100 as described herein. For example, the storage device 130 may be implemented on the computing device 200, via its hardware, software program, firmware, or a combination thereof. Although only one such computer is shown, for convenience, the computer functions relating to parameter configuration as described herein may be implemented in a distributed fashion on a number of similar platforms to distribute the processing load.

The computing device 200, for example, may include COM ports 250 connected to and from a network connected thereto to facilitate data communications. The computing device 200 may also include a processor (e.g., a processor 220), in the form of one or more processors (e.g., logic circuits), for executing program instructions. For example, the processor 220 may include interface circuits and processing circuits therein. The interface circuits may be configured to receive electronic signals from a bus 210, wherein the electronic signals encode structured data and/or instructions for the processing circuits to process. The processing circuits may conduct logic calculations, and then determine a conclusion, a result, and/or an instruction encoded as electronic signals. Then the interface circuits may send out the electronic signals from the processing circuits via the bus 210.

The computing device 200 may further include one or more storages configured to store various data files (e.g., program instructions) to be processed and/or transmitted by the computing device 200. In some embodiments, the one or more storages may include a high speed random access memory (not shown), a nonvolatile memory (not shown), a disk 270, a read-only memory (ROM) 230, a random-access memory (RAM) 240, or the like, or any combination thereof. In some embodiments, the one or more storages may further include a remote storage corresponding to the processor 220. The remote storage may connect to the computing device 200 via the network 140. The computing device 200 may also include program instructions stored in the one or more storages (e.g., the ROM 230, RAM 240, and/or another type of non-transitory storage medium) to be executed by the processor 220. The methods and/or processes of the present disclosure may be implemented as the program instructions. The computing device 200 may also include an I/O component 260, supporting input/output between the computing device 200 and other components. The computing device 200 may also receive programming and data via network communications.

Merely for illustration, only one processor is illustrated in FIG. 2. Multiple processors 220 are also contemplated; thus, operations and/or method steps performed by one processor 220 as described in the present disclosure may also be jointly or separately performed by the multiple processors. For example, if in the present disclosure the processor 220 of the computing device 200 executes both operation A and operation B, it should be understood that operation A and operation B may also be performed by two different processors 220 jointly or separately in the computing device 200 (e.g., a first processor executes operation A and a second processor executes operation B, or the first and second processors jointly execute operations A and B).

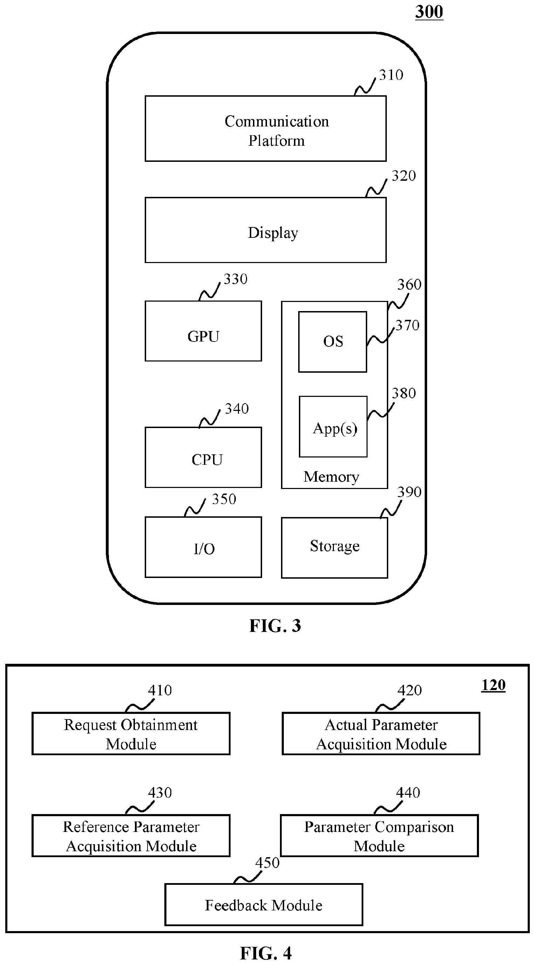

FIG. 3 is a schematic diagram illustrating exemplary hardware and/or software components of an exemplary mobile device according to some embodiments of the present disclosure. In some embodiments, the storage device 130 or the user device may be implemented on the mobile device 300.

As illustrated in FIG. 3, the mobile device 300 may include a communication platform 310, a display 320, a graphics processing unit (GPU) 330, a central processing unit (CPU) 340, an I/O 350, a memory 360, a mobile operating system (OS) 370, and a storage 390. In some embodiments, any other suitable components, including but not limited to a system bus or a controller (not shown), may also be in the mobile device 300.

In some embodiments, the mobile operating system 370 (e.g., iOS^{™}, Android^{™}, Windows Phone^{™}) and one or more applications 380 may be loaded into the memory 360 from the storage 390 in order to be executed by the CPU 340. The applications 380 may include a browser or any other suitable mobile apps for receiving and rendering information relating to parameter configuration or other information from the parameter configuration system 100. User interactions with the information stream may be achieved via the I/O 350 and provided to the storage device 130 and/or other components of the parameter configuration system 100 via the network 140.

FIG. 4 is a block diagram illustrating an exemplary processing device according to some embodiments of the present disclosure. The processing device 120 may include a request obtainment module 410, an actual parameter acquisition module 420, a reference parameter acquisition module 430, a parameter comparison module 440, and a feedback module 450.

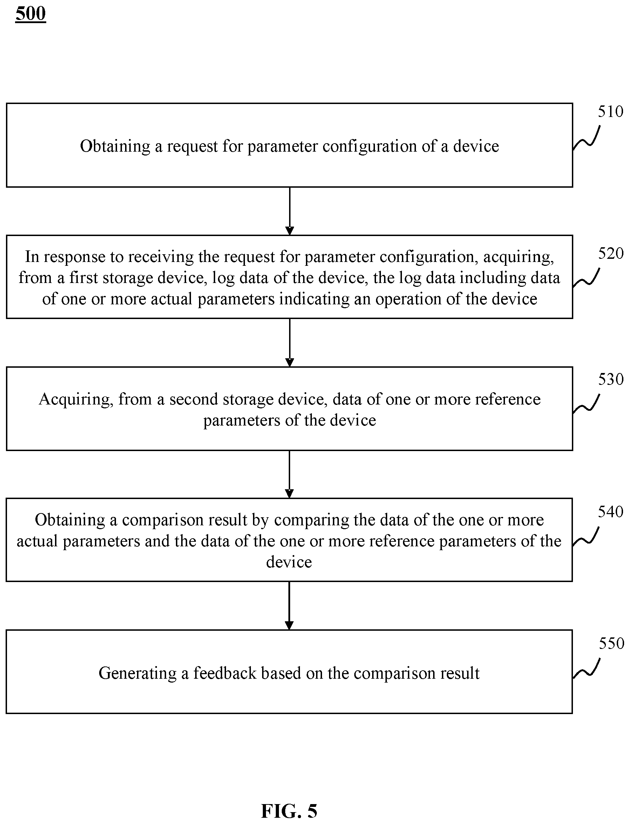

The request obtainment module 410 may be configured to obtain a request for parameter configuration of a device (e.g., the device 110), more descriptions of which may be found elsewhere in the present disclosure (e.g., FIGs. 5-10 or the descriptions thereof).

In response to receiving the request, the actual parameter acquisition module 420 may be configured to acquire, from a first storage device (e.g., the storage device 130), log data (e.g., including one or more actual parameters) of the device, more descriptions of which may be found elsewhere in the present disclosure (e.g., FIGs. 5-10 or the descriptions thereof).

The reference parameter acquisition module 430 may be configured to acquire, from a second storage device (e.g., the storage device 130), data of one or more reference parameters of the device, more descriptions of which may be found elsewhere in the present disclosure (e.g., FIGs. 5-10 or the descriptions thereof).

The parameter comparison module 440 may be configured to obtain a comparison result by comparing the data of the one or more actual parameters and the data of the one or more reference parameters of the device, more descriptions of which may be found elsewhere in the present disclosure (e.g., FIGs. 5-10 or the descriptions thereof).

The feedback module 450 may be configured to generate a feedback based on the comparison result, more descriptions of which may be found elsewhere in the present disclosure (e.g., FIGs. 5-10 or the descriptions thereof).

FIG. 5 is a flowchart illustrating an exemplary process for parameter configuration of a device according to some embodiments of the present disclosure. FIG. 6 is a schematic diagram illustrating exemplary parameter configuration of a device according to some embodiments of the present disclosure. In some embodiments, process 500 may be implemented as a set of instructions (e.g., an application) stored in the storage ROM 230 or RAM 240. The processor 220 and/or the modules in FIG. 4 may execute the set of instructions, and when executing the instructions, the processor 220 and/or the modules may be configured to perform process 500.

In 510, the processing device 120 (e.g., the request obtainment module 410) may obtain a request for parameter configuration of a device (e.g., the device 110 in FIG. 1). In some embodiments, the parameter configuration may include configuring one or more parameters which are used to facilitate monitoring an operation of the device (also referred to as monitoring parameters or configuration parameters). For example, the one or more parameters may be used to determine whether the operation of the device is normal.

In some embodiments, the one or more parameters may include parameters of at least one component of the device. In some embodiments, the device may include an imaging device, and the at least one component may include an imaging source, a detector, a cooling component, a target, a magnet, etc. In some embodiments, the device may include a treatment device, and the at least one component may include a treatment source, a detector (e.g., an electronic portal imaging device (EPID), a collimator, etc. In some embodiments, the device 110 may include a CT imaging device and exemplary parameters may include a parameter of an X-ray tube, a parameter of a target, a parameter of a detector, etc., of the CT imaging device.

In some embodiments, the device may include an MRI device. The operation of the MRI device may include scanning at least a portion of an object (e.g., a patient) to acquire imaging data of the object. The one or more parameters (e.g., as shown in FIG. 6) may include a cooling parameter, a gradient parameter (e.g., a temperature gradient), a magnet parameter (e.g., an intensity), the at least a portion of the object, a remaining disk capacity, or the like, or any combination thereof. In some embodiments, the magnet parameter may be of a main magnet used to generate a main field, be of a gradient coil used to generate a gradient field, be of a radio frequency (RF) coil used to generate an RF field, or the like, or any combination thereof. The cooling parameter may be of a cooling medium (e.g., helium) that is used to cool at least a portion (e.g., a main magnet) of the MRI device. For example, the cooling parameter may include a pressure, a flux, a temperature, etc., of the cooling medium. The gradient parameter may include a temperature gradient of at least a portion (e.g., a main magnet) of the MRI device.

In some embodiments, the request for parameter configuration may be generated manually or automatically. In some embodiments, the request for parameter configuration of the device may be generated based on an input of a user via a user interface implemented on a user terminal (e.g., the terminal device 150) (e.g., a mobile phone, a computer, a smart watch) in communication with at least one processor (e.g., the processing device 120, the device).

In 520, in response to receiving the request for parameter configuration, the processing device 120 (e.g., the actual parameter acquisition module 420) may acquire, from a first storage device (e.g., the storage device 130), log data of the device. In some embodiments, the log data may be associated with the operation of the device. For example, the log data may be generated in response to the operation of the device. In some embodiments, the acquisition of the data of the one or more actual parameters of the device may be performed automatically in response to receiving the request for parameter configuration.

In some embodiments, the log data of the device may include data of one or more actual parameters indicating the operation of the device. In some embodiments, the one or more actual parameters may be used to facilitate monitoring the operation of the device. In some embodiments, the one or more actual parameter may be used to determine whether to update one or more reference parameters of the device. In some embodiments, the one or more actual parameters may be used to determine whether the operation of the device is normal. In some embodiments, the processing device 120 may acquire the data of the one or more actual parameters of the device from the log data of the device.

In some embodiments, the device may include an MRI device. The operation of the MRI device may include scanning at least a portion of an object (e.g., a patient) to acquire imaging data of the object. The one or more actual parameters (e.g., as shown in FIG. 6) may include a cooling parameter, a gradient parameter (e.g., a temperature gradient), a magnet parameter (e.g., an intensity), the at least a portion of the object, a remaining disk capacity, or the like, or any combination thereof.

In some embodiments, data of each actual parameter may include a key of the actual parameter, a name of the actual parameter, whether the actual parameter is required to upload or the like, or any combination thereof. In some embodiments, the key of the actual parameter may be a unique identification of the actual parameter. In some embodiments, the processing device 120 may acquire the data of the one or more actual parameters from the first storage device based on one or more keys of the one or more actual parameters. In some embodiments, the acquisition of the data of the one or more actual parameters of the device may be performed automatically in response to receiving the request for parameter configuration.

In some embodiments, the first storage device may include at least one of a first database or a second database. The first database may be used to store log data of the device generated at a first time period. In some embodiments, the first time period may include a latest time period. The first database may be used to store latest log data of the device generated at the latest time period. In some embodiments, the latest time period may include a current time period, e.g., a current day. In some embodiments, the latest time period may include a predetermined time period before the current time period. For example, the first database may include a remote dictionary server (Redis) database, an Memcached database, MongoDB, RabbitMQ, Hazelcast, Cassandra, MySQL, SQLite, Aeropike, etc. In some embodiments, the second database may be used to store log data of the device generated at a second time period. The second time period may be before the first time period or include the first time period. For example, the second time period may include one or more days before a current day. As another example, the time range of the second time period may include the time range of the first time period. In some embodiments, the second database may include an Elasticsearch database, Datadog, Solr, Lucene, MongoDB, Algolia, Splunk, Kibana, Cassandra, etc.

In some embodiments, the processing device 120 may acquire the log data of the device from the first database or the second database. In some embodiments, the parameter configuration may be performed with respect to actual parameters generated at a third time period. The request for parameter configuration may include the third time period. The processing device 120 may acquire log data generated at the third time period from the first database or the second database for the parameter configuration. In some embodiments, if the first database includes log data generated at the third time period, the processing device 120 may acquire the log data from the first database. If the second database includes log data generated at the third time period, the processing device 120 may acquire the log data from the second database. If neither the first database nor the second database includes log data generated at the third time period, the processing device 120 may utilize log data generated in a fourth time period nearest to the third time period from the first database or the second database for the parameter configuration.

In some embodiments, the third time period may be the first time period, e.g., a current time period. The log data of the device may include latest log data at the current time period. The processing device 120 may determine whether the first database includes the latest log data at the current time period. In response to determining the first database includes the latest log at the current time period, the processing device 120 may acquire the log data from the first database. In response to determining the first database does not include the log data at the current time period, the processing device 120 may utilize log data generated at a time period nearest to the current time period from the second database for the parameter configuration.

In some embodiments, the log data or the one or more actual parameters may be represented in a language such as extensible markup language (XML) (e.g., as shown in FIG. 7), YAML, hypertext markup language (HTML), javascript object notation (JSON), Javascript, Python, hypertext preprocessor (PHP), Java, or the like, or any combination thereof.

In 530, the processing device 120 (e.g., the reference parameter acquisition module 430) may acquire, from a second storage device (e.g., the storage device 130), data of the one or more reference parameters of the device. The second storage device may be the same as or different from the first storage device. In some embodiments, the acquisition of the data of the one or more reference parameters may be performed automatically in response to receiving the request for parameter configuration.

In some embodiments, the one or more reference parameters may be used to facilitate monitoring the operation of the device. In some embodiments, the one or more reference parameters may be used to determine whether the operation of the device is normal. In some embodiments, the one or more reference parameters may be set according to a default setting of a parameter configuration system (e.g., the parameter configuration system 100) or provided by a user. In some embodiments, the one or more reference parameters may be determined using a machine learning model.

In some embodiments, the device may include an MRI device. The operation of the MRI device may include scanning at least a portion of an object (e.g., a patient) to acquire imaging data of the object. The one or more reference parameters may include a cooling parameter, a gradient parameter (e.g., a temperature gradient), a magnet parameter (e.g., an intensity), the at least a portion of the object, a remaining disk capacity, or the like, or any combination thereof.

In some embodiments, data of a reference parameter may include a key of the reference parameter, a name of the reference parameter, whether the reference parameter is required to upload, a lower limit of the reference parameter, an upper limit of the reference parameter, a show type (e.g., a curve, a table, a database) of the reference parameter, a permission version (e.g., including whether a user has the permission to modify the reference parameter) of the reference parameter, or the like, or any combination thereof.

In some embodiments, the second storage device may store a corresponding relationship (also referred to as a first relationship) between an identity of each of multiple devices and data of reference parameters of the each of the multiple devices. In some embodiments, the first relationship may be in form of a table, a curve, a database, a trained machine learning model, etc.

In some embodiments, the processing device 120 may obtain an identity of the device. The processing device 120 may also obtain the first relationship from the second storage device. The processing device 120 may determine the data of the one or more reference parameters based on the identity of the device and the first relationship. In some embodiments, the identity of the device may be a unique identification of the device. In some embodiments, the request for parameter configuration of the device may include the identity of the device. The processing device 120 may obtain the identity of the device from the request for parameter configuration.

In some embodiments, the second storage device may store a corresponding relationship (also referred to as a second relationship) between multiple devices in the same type and data of reference parameters of the multiple devices in the same type. In some embodiments, the second relationship may be in form of a table, a curve, a database, a trained machine learning model, etc.

In some embodiments, the processing device 120 may determine a type of the device. The processing device 120 may obtain the second relationship from the second storage device. The processing device 120 may determine the data of the one or more reference parameters based on the type of the device and the second relationship. In some embodiments, the device may include a medical device. For example, a type of the medical device may include MRI, X-ray, CT, PET, SPECT, linear accelerator, cyclotron, synchrotron, etc. In some embodiments, the type of the device may indicate a name and/or a key of a reference parameter.

In some embodiments, the processing device 120 may determine the type of the device based on the identity of the device. In some embodiments, the processing device 120 may determine the type of the device based on the identity of the device using a predetermined relationship between multiple devices of a same type and an identity of each of the multiple devices.

In some embodiments, the second storage device may store a corresponding relationship (also referred to as a third relationship) between multiple devices in the same mode and data of reference parameters of the multiple devices in the same mode. In some embodiments, the third relationship may be in form of a table, a curve, a database, a trained machine learning model, etc.

In some embodiments, the processing device 120 may determine a mode of the device. The processing device 120 may obtain the third relationship from the second storage device. The processing device 120 may determine the data of the one or more reference parameters based on the mode of the device and the third relationship. In some embodiments, the device may include an MR device. As shown in FIG. 8 and 9, exemplary modes of the MR device may include uMR 588, uMR 770, uMR 580, uMR 586, uMR 780, uMR 94T, uMR 560, etc. In some embodiments, the device may include a CT device. As shown in FIG. 6, exemplary modes of the CT device may include uCT 710, uCT 550, etc.

In some embodiments, the processing device 120 may determine the mode of the device based on the identity of the device. In some embodiments, the processing device 120 may determine the mode of the device based on the identity of the device using a predetermined relationship between multiple devices of a same mode and an identity of each of the multiple devices.

In some embodiments, the second storage device may store a corresponding relationship (also referred to as a fourth relationship) between an examination manner of each of multiple devices and data of reference parameters of each of the multiple devices. In some embodiments, the fourth relationship may be in form of a table, a curve, a database, a trained machine learning model, etc.

In some embodiments, the processing device 120 may determine an examination manner of the device. The processing device 120 may obtain the fourth relationship from the second storage device. The processing device 120 may determine the data of the one or more reference parameters based on the examination manner of the device and fourth relationship. In some embodiments, the device may include a CT device, and exemplary examination manners of the CT device may include a helical (or spiral) scan manner, an axial scan manner, etc.

In some embodiments, the processing device 120 may determine the examination manner of the device based on the type of the device. In some embodiments, the processing device 120 may determine the examination manner of the device based on the type of the device using a predetermined relationship between an examination manner of each of multiple devices and a type of each of the multiple devices.

In some embodiments, the processing device 120 may determine the examination manner of the device based on the identity of the device. In some embodiments, the processing device 120 may determine the examination manner of the device based on the identity of the device using a predetermined relationship between an examination manner of each of multiple devices and an identity of each of the multiple devices.

In some embodiments, the second storage device may store a corresponding relationship (also referred to as a fifth relationship) between feature information associated with each of multiple devices and data of reference parameters of the each of the multiple devices.

In some embodiments, the processing device 120 may obtain feature information associated with the device. The processing device 120 may also obtain the corresponding relationship from the second storage device. The processing device 120 may determine the data of the one or more reference parameters based on the feature information associated with the device and the corresponding relationship. In some embodiments, the feature information may include an identity of the device, a type of the device, a mode of the device, an examination manner of the device, a scanning parameter of an object (e.g., a patient), a parameter associated with a treatment plan of the object, or the like, or any combination thereof.

In some embodiments, the fifth relationship may be implemented by a parameter model. The processing device 120 may determine the data of the one or more reference parameters by inputting the feature information associated with the device into the parameter model. In some embodiments, the parameter model may be determined based on a machine learning technique. In some embodiments, the parameter model may be determined by training a preliminary model based on a plurality of training samples. For example, the device may include an MRI device, and the parameter model may be configured to determine a cooling parameter, a gradient parameter (e.g., a temperature gradient), a magnet parameter (e.g., an intensity), etc., of the MRI device. Each of the plurality of training samples may include training feature information of a training MRI device. One or more predetermined reference parameters (e.g., a cooling parameter, a gradient parameter (e.g., a temperature gradient), a magnet parameter (e.g., an intensity)) of the training MRI device may be a mask or ground truth corresponding to the training sample.

In 540, the processing device 120 (e.g., the parameter comparison module 440) may obtain a comparison result by comparing the data of the one or more actual parameters and the data of the one or more reference parameters of the device.

In some embodiments, types of the one or more actual parameters may be the same as or different from types of the one or more reference parameters. Taking FIG. 6 as an example, the types of the one or more reference parameters may include type 1, type 2, and type 3 while the types of the one or more actual parameters may include type 1, type 20, type 30, and type 4.

In some embodiments, a count of the one or more actual parameters may be the same as or different from a count of the one or more reference parameters. Still taking FIG. 6 as an example, the count of the one or more actual parameters may be 4 and the count of the one or more reference parameters may be 3.

In some embodiments, the processing device 120 may compare the data of the one or more actual parameters and the data of the one or more reference parameters of the device by matching the one or more actual parameters and the one or more reference parameters and determining a difference between the one or more actual parameters and the one or more reference parameters.

In some embodiments, the difference may include at least one of a type difference or a magnitude difference. In some embodiments, for a same type of the one or more reference parameters and the one or more actual parameters, the comparison result may include a magnitude difference between a reference parameter and an actual parameter of the same type. In some embodiments, for different types of the one or more reference parameters and the one or more actual parameters, the comparison result may include a type difference between the one or more reference parameters and the one or more actual parameters.

In some embodiments, the type difference may include a first type difference or a second type difference. In the first type difference, a type of an actual parameter may be different from each type of the one or more reference parameters. In other words, the actual parameter of the type may not match each of the one or more reference parameters. In the second type difference, a type of a reference parameter may be different from each type of the one or more actual parameters. In other words, the reference parameter of the type may not match each of the one or more actual parameters. Still taking FIG. 6 as an example, the first type difference may include type 20, type 30, and type 4. The second type difference may include type 2 and type 3.

In some embodiments, the difference between the one or more actual parameters and the one or more reference parameters may be presented in form of a table, a database, a curve, etc. In some embodiments, the table may include the one or more actual parameters, the one or more reference parameters, and one or more indexes indicating the difference. For example, as shown in FIG. 6, the one or more indexes may include a "match" index indicating a same type of the one or more actual parameters and the one or more reference parameters, a "no match" index for indicating the second type difference, an "add" index for indicating the first type difference, etc.

In some embodiments, the magnitude difference may include a difference between a magnitude of an actual parameter and a magnitude of a reference parameter that is the same type as the actual parameter. Still taking FIG. 6 as an example, the magnitude difference may include a difference between a magnitude of an actual parameter of type 1 and a magnitude of a reference parameter of type 1.

As described in connection with operation 520, the one or more actual parameters may be used to facilitate monitoring the operation of the device. In some embodiments, the one or more actual parameters may be used to determine whether to update the one or more reference parameters of the device. In some embodiments, the one or more actual parameters may be used to determine whether the operation of the device is normal.

In 550, the processing device 120 (e.g., the feedback module 450) may generate a feedback based on the comparison result. In some embodiments, the feedback may include adjusting the data of the one or more reference parameters, generating a reminder of an anomaly of the device, or the like, or any combination thereof. In some embodiments, the adjusting the data of the one or more reference parameters of the device may be performed by the processing device 120 or a user of the device.

In some embodiments, in response to determining that the comparison result indicates that the difference between the one or more actual parameters and the one or more reference parameter satisfies a condition (also referred to as a first condition), the processing device 120 may determine the feedback including adjusting the data of the one or more reference parameters of the device based on the difference between the one or more actual parameters and the one or more predetermined parameters.

In some embodiments, the first condition may include a first case, a second case, etc. In the first case, a type of an actual parameter may be different from each type of the one or more reference parameters. In the second case, a type of a reference parameter may be different from each type of the one or more actual parameters.

In some embodiments, for the first case, the adjusting the data of the one or more reference parameters of the device based on the difference between the one or more actual parameters and the one or more reference parameters may include updating the data of the one or more reference parameters by adding data of the actual parameter whose type is different from each type of the one or more reference parameters into the data of the one or more reference parameters. Still taking FIG. 6 as an example, data of the one or more reference parameters may be updated by adding data of actual parameters of type 20, type 30, and type 4 into the data of the one or more reference parameters. In some embodiments, for the second case, the adjusting the data of the one or more reference parameters of the device based on the difference between the one or more actual parameters and the one or more reference parameters may include removing the reference parameter whose type is different from each type of the one or more actual parameters. Still taking FIG. 6 as an example, data of the one or more reference parameters may be updated by removing data of reference parameters of type 2 and type 3. By doing this, an invalid reference parameter may be removed, and/or a missing reference parameter may be added, thereby improving the user experience and optimizing the resource utilization.

In some embodiments, the adjusting the data of the one or more reference parameters of the device based on the difference between the one or more actual parameters and the one or more predetermined parameters may include adjusting at least one relationship (e.g., the first relationship, the second relationship, the third relationship, the fourth relationship, the fifth relationship) as described in 530. For the removed reference parameter, at least one relationship corresponding to the removed reference parameter may be removed from the second storage device. For the added reference parameter, at least one relationship corresponding to the added reference parameter may be generated and stored in the second storage device.

In some embodiments, in response to determining that the comparison result indicates that the difference between the one or more actual parameters and the one or more reference parameter satisfies a condition (also referred to as a second condition), the processing device 120 may determine the feedback including a reminder that the operation of the device includes an anomaly. In some embodiments, the second condition may include the magnitude difference exceeding a threshold, an actual parameter exceeding a corresponding upper limit, an actual parameter smaller than a corresponding lower limit, or the like, or any combination thereof.

In some embodiments, in response to determining that the comparison result indicates that the difference between the one or more actual parameters and the one or more reference parameters does not satisfy a condition (e.g., the first condition, the second condition), the processing device 120 may determine the feedback including the data of the one or more reference parameters. Still taking FIG. 6 as an example, the processing device 120 may determine the feedback including data of a reference parameter of type 1. The processing device 120 may further monitor the device based on the data of the one or more reference parameters.

In some embodiments, after comparing the data of the one or more actual parameters and the data of the one or more reference parameters of the device the processing device 120 may mark the device with a label indicating that the comparison between the data of the one or more actual parameters and the data of the one or more reference parameters of the device has been obtained. After the comparison result is processed, the processing device 120 may remove the label of the device. In subsequent parameter configuration, the processing device 120 may not repeat performing the parameter configuration with respect to the marked device.

The parameter configuration may cause an extra load on at least one component (e.g., the device, the processing device 120, the first storage device, the second storage device) of the parameter configuration system. In order to avoid interfering other tasks of the at least one component of the parameter configuration system and/or efficiently perform the parameter configuration, in some embodiments, the request for parameter configuration of the device may be generated in response to determining that a current time is in a predetermined time period. The predetermined time period may include an idle time period when a load on each of the at least one component of the parameter configuration system is smaller than a threshold. In some embodiments, the threshold may be 50%, 75%, 90%, etc., of the maximum load of the component of the parameter configuration system. For example, the predetermined time period may include 22:00 - 03:00, 5:00 - 6:00, etc, in each day.

It should be noted that the above description is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure.

In some embodiments, operation 510 may be omitted. In some embodiments, the device or the processing device 120 may automatically generate the request for parameter configuration in response to determining that a current time is in the predetermined time period (e.g., an idle time period). In some embodiments, the device or the processing device 120 may automatically generate the request for parameter configuration periodically. In some embodiments, the device or the processing device 120 may automatically generate the request for parameter configuration in response to the initiation of the device.

In some embodiments, operation 520 may be performed before operation 510, and operation 510 may be performed, in response to the acquisition of the data of the one or more actual parameters, based on the identity of the device.

In some embodiments, operation 510 and operation 520 may be performed by the device. That is, the device may generate the request for parameter configuration and acquire the log data and/or the one or more actual parameters of the device.

In some embodiments, operation 520 and operation 530 may be performed simultaneously in response to receiving the request for parameter configuration of the device. In some embodiments, operation 530 may be performed before operation 520 in response to receiving the request for parameter configuration.

In some embodiments, the request for parameter configuration may include an instruction for reference parameter adjustment. The processing device 120 may adjust the one or more reference parameters based on the instruction for reference parameter adjustment. For example, the instruction for reference parameter adjustment may include adding a parameter of the device into the one or more reference parameters. As another example, the instruction for reference parameter adjustment may include removing a reference parameter from the one or more reference parameters. Further, the processing device 120 may obtain a comparison result by comparing the data of the one or more actual parameters and the data of the one or more adjusted reference parameters of the device.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "unit," "module," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB. NET, Python or the like, conventional procedural programming languages, such as the "C" programming language, Visual Basic, Fortran 2103, Perl, COBOL 2102, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be operably connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, for example, an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed object matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±1%, ±5%, ±10%, or ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. A method implemented on a computing device having at least one processor and at least one storage device, comprising:
obtaining a request for parameter configuration of a device;
**characterized by** further comprising:
in response to receiving the request,
acquiring, from a first storage device, log data of the device, the log data including data of one or more actual parameters indicating an operation of the device;
acquiring, from a second storage device, data of one or more reference parameters of the device;
obtaining a comparison result by comparing the data of the one or more actual parameters and the data of the one or more reference parameters of the device; and
generating a feedback based on the comparison result.

2. The method of claim 1, wherein the second storage device stores a corresponding relationship between an identity of each of multiple devices and data of reference parameters of the each of the multiple devices, and
the acquiring the data of the one or more reference parameters of the device includes:
obtaining an identity of the device;
obtaining the corresponding relationship from the second storage device; and
determining the data of the one or more reference parameters based on the identity of the device and the corresponding relationship.

3. The method of claim 1, wherein the second storage device stores a corresponding relationship between multiple devices in the same type and data of reference parameters of the multiple devices in the same type, and
the acquiring the data of the one or more reference parameters of the device includes:
determining a type of the device;
obtaining the corresponding relationship from the second storage device; and
determining the data of the one or more reference parameters based on the type of the device and the corresponding relationship.

4. The method of claim 1, wherein the second storage device stores a corresponding relationship between feature information associated with each of multiple devices and data of reference parameters of the each of the multiple devices, and
the acquiring the data of the one or more reference parameters of the device includes:
obtaining feature information associated with the device;
obtaining the corresponding relationship from the second storage device; and
determining the data of the one or more reference parameters based on the feature information associated with the device and the corresponding relationship.

5. The method of claim 4, wherein the corresponding relationship is implemented by a parameter model, and
the determining the data of the one or more reference parameters based on the feature information associated with the device and the corresponding relationship includes:
determining the data of the one or more reference parameters by inputting the feature information associated with the device into the parameter model.

6. The method of any one of claims 1-5, wherein the obtaining the comparison result by comparing the data of the one or more actual parameters and the data of the one or more reference parameters of the device includes:
determining a difference between the one or more actual parameters and the one or more reference parameters, the difference including at least one of a type difference or a magnitude difference.

7. The method of claim 6, wherein the generating the feedback based on the comparison result includes:
in response to determining that the comparison result indicates that the difference between the one or more actual parameters and the one or more reference parameters satisfies a condition, determining the feedback including
adjusting the data of the one or more reference parameters of the device based on the difference between the one or more actual parameters and the one or more predetermined parameters.

8. The method of claim 7, wherein the difference between the one or more actual parameters and the one or more reference parameters satisfying the condition includes at least one of a first case or a second case, in the first case, a type of an actual parameter is different from each type of the one or more reference parameters, and in the second case, a type of a reference parameter is different from each type of the one or more actual parameters.

9. The method of claim 8, wherein the adjusting the data of the one or more reference parameters of the device based on the difference between the one or more actual parameters and the one or more predetermined parameters includes:
updating the data of the one or more reference parameters by adding data of the actual parameter whose type is different from each type of the one or more reference parameters into the data of the one or more reference parameters; or
updating the data of the one or more reference parameters by removing the reference parameter whose type is different from each type of the one or more actual parameters.

10. The method of any one of claims 6-9, wherein the generating the feedback based on the comparison result includes:
in response to determining that the comparison result indicates that the difference between the one or more actual parameters and the one or more reference parameters satisfies a condition, determining the feedback including a reminder that the operation of the device includes an anomaly.

11. The method of any one of claims 6-10, wherein the generating the feedback based on the comparison result includes:
in response to determining that the comparison result indicates that the difference between the one or more actual parameters and the one or more reference parameter does not satisfy a condition, determining the feedback including the data of the one or more reference parameters; and the method further include:
monitoring the device based on the data of the one or more reference parameters.

12. The method of any one of claims 1-11, wherein
the first storage device includes at least one of a first database or a second database;
the first database is used to store latest log data of the device; and
the second database is used to store log data of the device generated at a time period.

13. The method of claim 12, wherein the acquiring the log data of the device includes:
acquiring the log data of the device from the first database or the second database, the log data includes latest log data at a current time period.

14. An apparatus, comprising:
at least one storage device including a set of instructions;
at least one processor in communication with the at least one storage device, wherein when executing the set of instructions, the at least one processor is configured to perform a method comprising:
obtaining a request for parameter configuration of a device;
**characterized by** further comprising:
in response to receiving the request,
acquiring, from a first storage device, log data of the device, the log data including data of one or more actual parameters indicating an operation of the device;
acquiring, from a second storage device, data of one or more reference parameters of the device;
obtaining a comparison result by comparing the data of the one or more actual parameters and the data of the one or more reference parameters of the device; and
generating a feedback based on the comparison result.

15. A non-transitory computer readable medium, comprising at least one set of instructions, wherein when executed by at least one processor of a computing device, the at least one set of instructions cause the at least one processor to effectuate a method of any one of claims 1-13.
